# FASCICULE DE BREVET EUROPEEN

(11) **EP 2 696 827 B1**
(45) Date de publication et mention de la délivrance du brevet: **31.05.2017**
(21) Numéro de dépôt: 12722416.0
(22) Date de dépôt: 13.04.2012
(51) Int. Cl.: A61F 13/02

(54) **PANSEMENT ABSORBANT HYDROCELLULAIRE, SES UTILISATIONS POUR LE TRAITEMENT DES PLAIES CHRONIQUES ET AIGUËS**
HYDROZELLULARER SAUGFÄHIGER WUNDVERBAND UND SEINE VERWENDUNG ZUR BEHANDLUNG VON CHRONISCHEN UND AKUTEN WUNDEN
HYDROCELLULAR ABSORBENT DRESSING, AND USES THEREOF FOR THE TREATMENT OF CHRONIC AND ACUTE WOUNDS

(30) Priorité: 15.04.2011 FR 1153325
(43) Date de publication de la demande: 19.02.2014
(73) Titulaire: URGO RECHERCHE INNOVATION ET DEVELOPPEMENT, 21300 Chenôve (FR)
(72) Inventeur: AUGUSTE, Stéphane, F-21490 Varois Et Chaignot (FR); PERNOT, Jean Marc, F-21000 Dijon (FR); DANEROL, Anne-Sophie, F-21000 Dijon (FR); CHARRE, Aurélie, F-69250 Montanay (FR)
(74) Mandataire: Chantraine, Sylvie Hélène
(86) Numéro de dépôt international: PCT/FR2012/050811
(87) Numéro de publication internationale: WO 2012/140377

(56) Documents cités:
- WO-A1-96/01659
- WO-A1-2004/064879
- WO-A1-2008/146529
- WO-A1-2010/147533
- WO-A2-2008/012443
- US-A1- 2009 216 168

## Description

La présente invention se rapporte à un pansement comprenant un support de protection imperméable respirant et une mousse absorbante, ainsi qu'à son utilisation pour le soin des plaies chroniques, comme les ulcères, les escarres, ou aiguës comme les brûlures.

Les pansements absorbants hydrocellulaires utilisés pour le traitement des plaies sont généralement constitués de l'assemblage d'une mousse absorbante et d'un support de protection imperméable et respirant. Ces pansements peuvent comprendre une bordure adhésive qui permet de les fixer sur la peau saine qui entoure la plaie. Ils peuvent également comprendre une couche d'interface positionnée sur la mousse absorbante, pour éviter le contact direct entre la mousse et la plaie.

Par exemple, le pansement absorbant Cellosorb^{®} Contact Adhésive comprend un support protecteur non tissé en polyuréthane élastique de haute respirabilité, une mousse de polyuréthane absorbante disposée au centre du support, et une couche d'interface discontinue qui recouvre la mousse. L'interface facilite la pose et le retrait atraumatique du pansement grâce à ses propriétés de micro-adhérence. Le produit Cellosorb^{®} Contact Adhésive comprend un trottoir recouvert d'un adhésif acrylique. Or, les adhésifs acryliques appliqués sur la bordure des pansements présentent une tolérance non optimale pour la peau périlésionnelle particulièrement fragile dans le cas des plaies chroniques.

Il est préférable d'utiliser, dans le soin des plaies chroniques et aiguës, des pansements dont le trottoir est recouvert d'un adhésif siliconé, lequel est plus respectueux de la peau périlésionnelle. Les adhésifs siliconés sont cependant totalement occlusifs si bien qu'il est nécessaire de les appliquer de façon discontinue pour assurer la bonne respirabilité du pansement. Or, l'application discontinue de l'adhésif siliconé a pour conséquence de trop diminuer son pouvoir d'adhésion aux autres matériaux constituant le pansement, en particulier aux matériaux absorbants, et plus particulièrement en milieu humide.

La réalisation de pansements absorbants qui utilisent un adhésif siliconé doit donc remplir un cahier des charges complexe et concilier des caractéristiques contradictoires. Les principaux critères que doivent vérifier le pansement sont essentiellement de présenter une bonne respirabilité tout en évitant les risques de fuites et de macération, d'être imperméable aux liquides et aux bactéries, d'être respirant (c'est-à-dire perméable à la vapeur d'eau), de garder sa cohésion quand on le retire, et d'être facile à fabriquer.

Le pansement doit également être facile à poser et rester en place au cours du temps le plus longtemps possible sans altérer la peau périlésionnelle, présenter une capacité d'absorption élevée, et ne pas altérer la cicatrisation de la plaie lors de son retrait. Le pansement doit aussi épouser la morphologie du patient et être compatible avec l'utilisation complémentaire d'un système de contention. Il doit aussi être le plus souple possible, et ne pas se rigidifier au cours de l'absorption des exsudats de la plaie.

Le choix des matériaux qui constituent le pansement, l'agencement des matériaux les uns par rapport aux autres, et les moyens d'assemblage de ces derniers sont très complexes pour obtenir toutes ces propriétés à la fois.

La seule solution proposée jusqu'à présent pour obtenir un pansement comprenant un adhésif siliconé, imperméable, respirant et cohésif, a consisté à faire réticuler le gel de silicone adhésif sur une seule face d'un film de polyuréthane perforé, en conservant les perforations du film ouvertes. Le support enduit d'adhésif siliconé est fixé au support du pansement avec un adhésif acrylique ou par thermoscellage.

Dans le produit Mepilex^{®} Border par exemple, le film de polyuréthane perforé enduit de gel de silicone adhésif est utilisé comme couche d'interface. Une couche discontinue de gel de silicone adhésif recouvre donc à la fois la mousse absorbante préalablement placée au centre du film support, et le trottoir du support destiné à adhérer à la peau. De la même façon, dans la demande WO 2010/147533 une couche discontinue d'adhésif silicone recouvre une mousse de polyuréthane absorbante et le support. Cette couche discontinue est constituée d'un film de polyuréthane perforé enduit d'un seul côté de gel de silicone. Cette structure discontinue permet d'éviter d'avoir à fixer le gel de silicone une fois réticulé à la couche absorbante du pansement. En effet, le gel de silicone une fois réticulé ne peut être durablement fixé à une mousse absorbante couramment utilisée dans la fabrication d'un pansement.

Cependant, dans cette structure de pansement, le gel de silicone est en contact direct avec la plaie. Le fait que l'adhésif siliconé recouvre l'intégralité de la surface du pansement ne permet pas d'adapter de façon spécifique la couche d'interface qui peut recouvrir la surface de la mousse absorbante; c'est pourquoi il a été proposé dans le document US 2010/0292626, un pansement dans lequel seul le trottoir est recouvert d'adhésif siliconé, et la mousse absorbante est en contact direct avec la plaie. Pour réaliser cette structure, un film de polyuréthane enduit de gel de silicone est découpé à la forme du trottoir, puis il est collé à un film support avec un adhésif acrylate. Dans ce pansement, le film de polyuréthane est recouvert d'une couche de gel de silicone continue tandis que l'adhésif acrylate est enduit de façon discontinue. La réalisation de ce pansement est cependant complexe à cause des étapes de découpe et de centrage des différents constituants.Un autre exemple est le pansement du document WO2008146529. Il existe donc un besoin de fabriquer un pansement imperméable aux liquides et de haute perméabilité à la vapeur d'eau qui utilise un adhésif siliconé, lequel pansement est plus facile à fabriquer que les pansements de l'art antérieur de ce type, et reste cohésif quand on le retire de la plaie.

La Demanderesse a trouvé un nouveau moyen de fixer une mousse absorbante à un support enduit d'un gel de silicone qui permet d'obtenir un niveau de cohésion des différents matériaux assemblés suffisant quand le pansement est sec mais aussi quand il est chargé des exsudats de la plaie. Le pansement est également plus facile à fabriquer que les pansements de l'art antérieur qui utilisent des adhésifs siliconés. Grâce à ce pansement, il n'est pas obligatoire de recouvrir toute la surface du pansement avec le gel de silicone adhésif pour simplifier le procédé de fabrication du pansement, si bien qu'une autre couche d'interface plus respectueuse de la plaie peut éventuellement être appliquée sur la mousse.

L'invention a ainsi pour objet un pansement comprenant une mousse absorbante et un support de protection imperméable aux fluides et perméable à la vapeur d'eau,
- ledit support étant constitué par l'assemblage d'un film continu et d'une armature ajourée enduite, sur au moins une de ses faces, d'un gel de silicone adhésif sans obturer les ouvertures de l'armature, ladite armature recouvrant l'intégralité de la surface du film ,
- ledit pansement comprenant en outre un voile non absorbant intercalé entre ladite mousse absorbante et ledit support, lequel voile colle au gel de silicone adhésif enduit sur ladite armature, et fixé à la mousse absorbante, de préférence seulement sur sa périphérie.

Le voile non absorbant est un élément essentiel de l'invention car un support enduit d'un gel de silicone sur toute la surface d'une de ses faces, et en particulier de façon discontinue, ne colle pas à une mousse absorbante. La mousse ne peut pas non plus être assemblée, à l'aide d'un adhésif acrylate, au support enduit de gel de silicone.

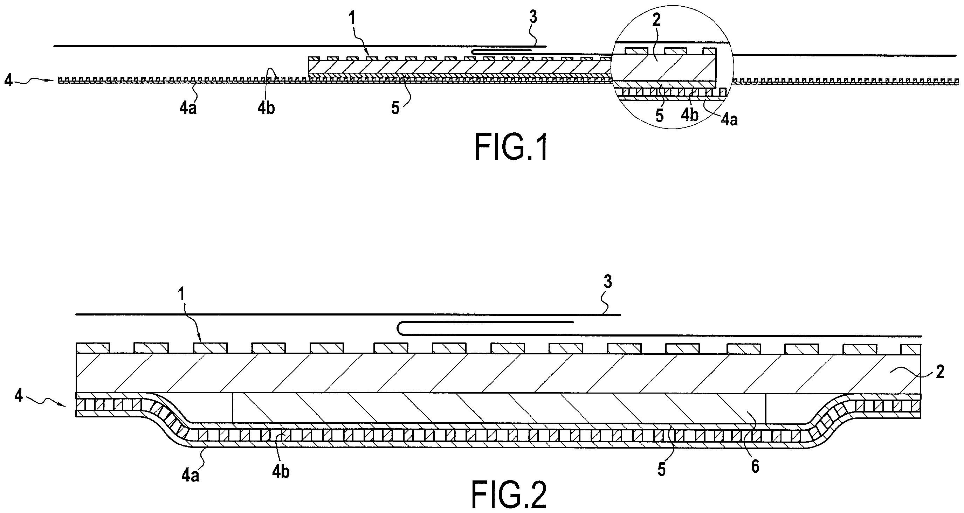

De tels pansements sont par exemple décrits par des vues schématiques dans les figures 1 à 3 en annexe. Dans ces figures, le pansement est représenté en coupe droite.

Sur la figure 1, une mousse absorbante 2 est recouverte, du côté faisant face à la plaie, d'une couche d'interface discontinue 1 protégée par un protecteur pelable 3.

Le support 4 est constituée par l'assemblage d'un film imperméable aux liquides et perméable à la vapeur d'eau 4a, et d'une armature ajourée enduite d'un gel de silicone adhésif 4b sans obturer les ouvertures de l'armature. Le support 4 est fixé à la mousse absorbante 2 par l'intercalage d'un voile non absorbant 5.

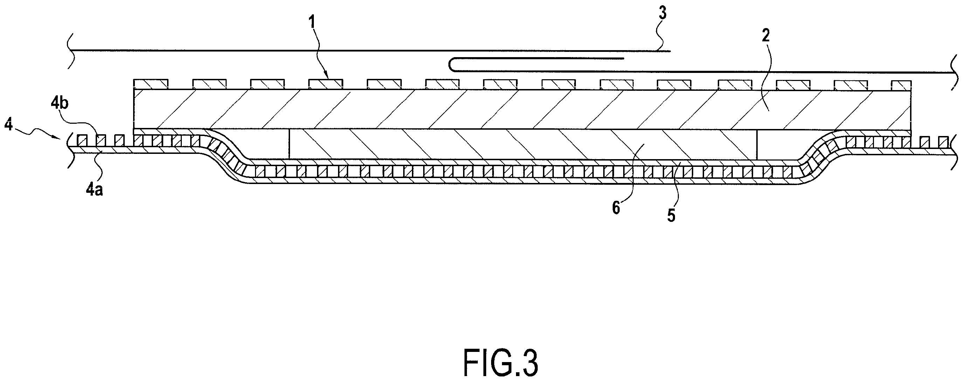

Sur les figures 2 et 3, un non tissé absorbant 6 est intercalé entre le voile 5 et la mousse absorbante 2. Le voile 5 est fixé sur sa périphérie à la mousse absorbante 2 de manière à emprisonner le non tissé absorbant 6 dont la dimension est inférieure à celle de la mousse absorbante.

Dans la figure 2, le support a la même taille que la mousse absorbante, tandis que dans les figures 1 et 3, le support est plus large que la mousse absorbante et le voile non absorbant de manière à former des marges ou des bordures adhésives qui peuvent adhérer à la peau.

Le support est de préférence imperméable aux fluides et aux micro-organismes pathogènes extérieurs tout en assurant une perméabilité à la vapeur d'eau, de manière à éviter à la fois le contact de la plaie avec des liquides extérieurs et des bactéries et la macération de la plaie. On parle alors de « support imperméable et respirant ».

Le support est de préférence mince et souple, de manière à mieux épouser la forme du corps et suivre les mouvements sans risquer de se détacher. Le support est avantageusement conformable. Son épaisseur peut être comprise entre 100 et 600 µm, de préférence entre 250 et 500 µm.

Le support faisant partie du pansement selon l'invention est constitué d'un film continu 4a et d'une armature ajourée enduite d'un gel de silicone adhésif 4b sans obturer les ouvertures de l'armature. Le film est continu en ce sens qu'il n'a pas subi une étape de perforation.

Le film peut être remplacé par un complexe mousse/film ou un complexe textile/film. Parmi les films utilisables, on peut citer à titre d'exemple les films en polyétheruréthane, en polyétheramide, ou en polyétherester.

L'épaisseur du film est par exemple comprise entre 5 et 200 microns, de préférence entre 10 et 75 microns, de préférence encore entre 10 et 50 microns.

Le film continu est imperméable aux liquides et perméable à la vapeur d'eau. Le film continu peut être un film ou un complexe cité précédemment s imperméable respirant tels ceux utilisés de façon courante pour la fabrication des pansements absorbants. Le film présente avantageusement un taux de transmission de vapeur d'eau (MVTR : Moisture Vapor Transmission Rate) supérieur à 3 000 g/m²/24 heures, de préférence supérieur ou égal à 7 000 g/m²/24 heures, de préférence encore supérieur ou égal à 10 000 g/m²/24 heures. L'armature ajourée est enduite par un composé silicone sans obturer les ouvertures de l'armature. Cette armature ainsi enduite est avantageusement choisie de manière à ce que la valeur du taux de transmission de vapeur d'eau du support reste satisfaisante, notamment supérieure ou égale à 4 000 g/m²/24 heures, de préférence supérieure ou égal à 5 000 g/m²/24 heures. Une technique de mesure du taux de transmission de vapeur d'eau en contact liquide est décrite dans la norme NF-EN 13726-2 (Chapitre 3.3).

Lorsque le pansement comporte un trottoir, l'armature permet de rigidifier le support, de manière à ce qu'il ne s'enroule pas sur lui-même après retrait d'un protecteur pelable éventuellement utilisé pour protéger la mousse ou la couche d'interface, et/ou la bordure adhésive.

L'armature pourra être constituée de tout matériau ajouré tels un film perforé, un filet thermoplastique, un textile comme par exemple un tissé, un tricot, ou un non tissé, de préférence élastique pour une meilleure tenue du pansement sur la peau. Un film perforé sera par exemple en polyéthylène ou en polypropylène. Un textile tissé sera par exemple en polyéthylène téréphtalate ou en polyamide. Le grammage de l'armature est de préférence compris entre 10 et 500 g/m², par exemple entre 20 et 300 g/m². L'armature peut être enduite de gel de silicone sur une de ses faces, sur ses deux faces, voir sur toute sa surface. La taille des ouvertures de l'armature peut être comprise entre 0,1 et 5 mm, par exemple entre 0,5 et 3 mm. La surface ouverte de l'armature représente de préférence de 1 à 99%, de préférence de 25 à 90%, de préférence encore de 30 à 80%, de la surface du film continu, et la surface ouverte de l'armature une fois recouverte de gel de silicone représente de préférence de 10 à 99%, de préférence de 10 à 60%, de préférence encore de 25 à 75% de la surface du film continu.

Selon un mode de mise en oeuvre, on utilisera un tricot, de préférence un tricot enduit de gel de silicone sur toute sa surface sans obturer les ouvertures du tricot. L'ensemble de la masse du tricot est enrobée de gel de silicone, par exemple par trempage du tricot dans un mélange précurseur du gel de silicone.

Selon un autre mode de réalisation, l'armature est un film perforé enduit de gel de silicone sur une seule de ses faces sans obturer les perforations du film, par exemple un film de polyuréthane perforé, qui pourra être fixé au film continu par la chaleur, les ultrasons, la haute fréquence ou un adhésif.

Le gel de silicone adhésif est un composé de silicone dont la structure est réticulée. Le gel de silicone présente une cohésion telle qu'il ne laisse pas de résidus sur la peau et reste accroché à l'armature quand on retire le pansement. Il peut être fabriqué à partir de précurseurs de silicones qui réticulent après leur mise en contact suivant une réaction d'hydrosilylation ou de condensation. De tels systèmes sont connus de l'art antérieur, par exemple dans les documents EP-A-0 251 810, EP-A-0 300 620 ou US-A-4 921 704. Les mélanges de précurseurs décrits dans ces documents comprennent pour l'essentiel :
- un composant A qui comprend au moins une polydiméthylsiloxane substituée par un groupe vinyle à chacune de ses extrémités, et un catalyseur de platine, et
- un composant B de polydiméthylsiloxane qui comprend au moins deux groupes hydrogénosilanes.

La mise en présence des deux composants provoque une réaction de réticulation (crosslinking reaction) des deux polydiméthylsiloxanes fonctionnalisées qui se produit avantageusement à température ambiante et peut être accélérée par la chaleur.

Des additifs comme des pigments, des inhibiteurs ou des charges de remplissage peuvent être incorporés à l'un au moins des deux composants.

Les précurseurs du gel de silicone adhésif peuvent être choisis parmi les produits suivants : Silbione RT Gel^{®} 4712 A&B et Silbione RT Gel^{®} 4717 A&B de Bluestar Silicones, Wacker Silgel^{®} 612 de Wacker-Chemie GmbH, Nusil^{®} MED-6340, Nusil^{®} MED-6345, Nusil^{®} MED3-6300, ou Nusil^{®} MED12-6300 de Nusil Technology, et D-7-9800^{®} de Dow Corning.

Le gel de silicone est de préférence choisi de telle façon que le support présente un pouvoir adhésif sur peau, selon la méthode EN 1939, supérieur à 40 cN/cm, et préférence 45 cN/cm. Un échantillon de support de 20 mm de large et 150 mm de long est posé sur l'avant-bras. Au bout de 10 minutes, on mesure le pouvoir adhésif avec un dynamomètre à une vitesse de traction de 900 mm/min avec un angle de 90°.

Le gel de silicone est de préférence appliqué sur l'armature ajourée sans obturer les ouvertures de l'armature à raison d'un grammage compris entre 100 et 500 g/m², de préférence entre 150 et 250 g/m², de manière à assurer un compromis entre un taux de transmission de vapeur d'eau suffisant et une adhésion au film continu ou à la peau suffisante.

A titre d'exemple, on peut utiliser un support constitué de l'association d'un film en polyuréthane de 30 µm d'épaisseur qui présente un taux de transmission de vapeur d'eau de l'ordre de 7000 g/m²/24 heures, et d'un tricot de polyester 40 g/m² enduit d'un gel de silicone sur toute sa surface à raison de 200 g/m². Ce support présente une épaisseur de l'ordre de 400 µm et une MVTR de l'ordre de 5000 g/m²/24 heures.

Dans un mode mise en oeuvre, la taille du support est supérieure à celle de la mousse absorbante et la mousse est centrée sur le support de manière à créer des bordures adhésives.

Le pansement selon l'invention comprend un voile intercalé entre la mousse absorbante et le support, et destiné à les assembler. Ce moyen de fixation est nécessaire car la surface du support enduite d'un gel de silicone adhésif n'adhère pas de façon suffisante à la mousse absorbante, en particulier en milieu humide.

Le voile intercalé entre la mousse absorbante et le support est un non tissé non absorbant de faible grammage. Le non tissé peut être tout type de non tissé couramment utilisé dans le domaine de l'hygiène et des pansements, notamment un non tissé filé lié (spun laid), cardé (carded) ou hydrolié (spun lace). Son grammage est de préférence compris entre 15 et 50 g/m², de préférence entre 20 et 40 g/m².

Le voile est non absorbant en ce sens qu'il ne contient pas de fibres absorbantes telles que la rayonne, la viscose, les dérivés de cellulose, et qu'il ne contient pas de particules absorbantes.

Il pourra comprendre des fibres de polyamide, de polyester, de polyuréthane et ou de polyoléfines. Selon un mode de réalisation, le voile comprend des fibres de polyéthylène. Les fibres peuvent être monocomposants, ou bicomposants de type coeur/écorce ou côte-à-côte. On choisira par exemple un non tissé spun laid, de préférence de type spunbond.

Le voile non absorbant sera de préférence constitué de fibres hydrophobes, mais il pourra aussi être constitué de fibres hydrophiles et avoir subi un traitement pour le rendre hydrophobe. Le voile peut être constitué de plusieurs couches, dans la mesure où sa porosité est suffisante, la couche venant au contact du gel de silicone adhésif étant non absorbante et de préférence hydrophobe.

Le voile non absorbant est fixé à la mousse absorbante sur toute sa surface, ou de préférence seulement sur sa périphérie par les technologies classiques de fixation telles la chaleur, les ultrasons, la haute fréquence, ou avec des adhésifs.

Dans le cadre de la présente invention, on utilisera par exemple un non tissé spun bond constitué de fibres de polyéthylène présentant un grammage compris entre 30 et 40 g/m², tel que celui commercialisé par la société Freudenberg sous la dénomination Vilmed^{®} LSO 1040 WEISS.

Dans le cadre de la présente invention, la mousse absorbante peut être choisie parmi les mousses absorbantes hydrophiles et les mousses absorbantes hydrophobes rendues absorbantes par l'incorporation de particules absorbantes.

Des mousses absorbantes hydrophiles et leur procédé de fabrication, bien connus de l'homme de l'art, sont décrits dans les demandes de brevet WO 96/16099, WO 94/29361, WO 93/04101, EP 420 515, EP 392 788, EP 299 122, et WO 2004/074343. De telles mousses correspondent par exemple à la référence MCF03 commercialisée par la société AMS, ou à la référence 562-B commercialisée par la société RYNEL.

Selon une version préférée de la présente invention, on utilisera une mousse de polyuréthane hydrophile dont l'épaisseur est choisie en fonction de la capacité d'absorption souhaitée. Dans le cadre de la présente invention, on préférera tout particulièrement une mousse de polyuréthane de 1,5 à 5 mm d'épaisseur.

Si l'on souhaite augmenter la capacité d'absorption du pansement, on pourra associer à la mousse décrite précédemment une deuxième couche absorbante choisie parmi les mousses, les textiles absorbants, comme par exemple les textiles tissés ou tricotés, les textiles non tissés.

Selon une version de la présente invention, on utilisera l'association d'une mousse 2, par exemple une mousse polyuréthane hydrophile, et d'un non tissé absorbant 6. Le non tissé absorbant est de préférence placé entre la mousse absorbante et le voile, comme représenté sur les figures 2 et 3. Selon cette variante, le non tissé absorbant a de préférence des dimensions inférieures à celles de la mousse afin d'emprisonner le non tissé absorbant dans une enveloppe constituée du voile et de la mousse dont les bords sont fixés.

Le non tissé absorbant est préférablement constitué de fibres absorbantes telles que des fibres de cellulose, de rayonne ou de viscose. Le non tissé est préférablement obtenu par la méthode de fabrication par voie sèche connue sous le nom de voie aérodynamique ou "airlaid".

Tous les modes de liages couramment utilisés dans cette technologie pourront être employés pour le non tissé de l'invention : liage par enduction spray de latex, liage par incorporation de fibres ou de poudres thermoliantes puis traitement thermique, liage par association de ces deux techniques, liage par simple compression des fibres. Ce dernier mode de liage, ne faisant pas appel à l'incorporation de matériaux thermoliants ou de latex, sera préféré.

Selon un mode de mise en oeuvre de la présente invention, le non tissé incorpore des particules de polymères super-absorbants dans une proportion comprise entre 1 et 70% en poids, de préférence de 25 à 55% en poids, du poids total du non tissé. Le polymère super-absorbant peut être choisi parmi les polymères acryliques dont leurs sels tels que les polyacrylates de sodium.

Selon un mode préféré de la présente invention on utilisera un non tissé à base de particules de polymères superabsorbants et de fibres de cellulose sans l'incorporation de matériaux thermoliants ou de latex, qui sera recouvert sur chacune de ces faces par un voile cellulosique. Selon une autre variante de la présente invention on pourra aussi employer comme non tissé absorbant un non tissé constitué de deux voiles cellulosiques entre lesquels sont incorporés des particules de polymères super-absorbants seules ou en association avec des liants.

Le non tissé absorbant peut également comprendre des fibres superabsorbantes.

Dans le cadre de la présente invention, on préfère utiliser un non tissé présentant une épaisseur comprise entre 0,5 et 3 mm, et/ou un grammage compris entre 200 et 500 g/m² et/ou une absorption supérieure à 5000 g/m², de préférence encore supérieure ou égale à 7000 g/m². L'absorption pourra être mesurée selon la norme EDANA 440.1.99.

Des non tissés absorbants appropriés sont par exemple commercialisés par la société EAM Corporation sous la référence Novathin^{®}.

On pourra avantageusement déposer sur la surface de la mousse absorbante, faisant face à la plaie une couche d'interface discontinue 1 destinée à entrer en contact avec la plaie. La couche d'interface est discontinue pour permettre l'accès des exsudats à la mousse absorbante ; elle n'altère pas la plaie lors du retrait du pansement.

Selon un mode de réalisation de l'invention, le pansement ne comprend pas de trottoir et la couche d'interface est micro-adhérente à la plaie, c'est-à-dire qu'elle permet de fixer provisoirement le pansement sur la plaie, et celui-ci peut être retiré sans que la structure de la plaie ou de la peau périlésionnelle soit altérée, si bien que ce pansement est repositionnable et facilite les soins infirmiers. Cette fixation peut aider le personnel soignant ou l'utilisateur à recouvrir le pansement d'un moyen de contention, d'un ruban adhésif, d'une bande ou d'un filet de maintien.

La composition de la couche d'interface peut être hydrophobe, hydrophile ou amphiphile.

On peut ainsi citer des compositions à base de polymères silicone en particulier des gels de silicone, des gels de polyuréthane, des compositions à base d'élastomère incluant des hydrocolloïdes, voire des hydrogels comme par exemple des hydrogels à base de poly(AMPS).

Dans le cadre de la présente invention, on préférera tout particulièrement utiliser une couche discontinue de composition contenant un élastomère, un plastifiant et des hydrocolloïdes. Cette couche d'interface favorise le processus de cicatrisation en maintenant un environnement humide au niveau de la plaie, et permet également de véhiculer des actifs, ce qui n'est pas le cas des interfaces siliconées.

L'élastomère peut être choisi parmi les polymères séquencés poly(styrène-oléfine-styrène) triblocs éventuellement associés à des copolymères diblocs. Les copolymères triblocs peuvent être des copolymères séquencés poly(styrène-éthylène-butylène-styrène) (en abrégé SEBS) vendus sous la dénomination KRATON^{®} G1651, KRATON^{®} G1654 ou KRATON^{®} G1652, ou des copolymères séquencés poly(styrène-éthylène-propylène-styrène) (en abrégé SEPS).

Parmi les composés plastifiants susceptibles d'être utilisés, on peut citer en particulier les huiles minérales, les polybutènes ou encore des dérivés de phtalate. D'une façon particulièrement préférée, on utilisera une huile plastifiante minérale choisie parmi les produits commercialisés sous les dénominations ONDINA^{®}933 et ONDINA^{®}919.

Comme hydrocolloïdes appropriés, on peut citer par exemple la pectine, les alginates, les gommes végétales naturelles comme en particulier la gomme de Karaya, les dérivés de cellulose tels que les carboxyméthylcelluloses et leurs sels de métal alcalin tels que le sodium ou le calcium connus sous la référence CMC Blanose^{®} 7H4XF, ainsi que les polymères synthétiques à base de sels de l'acide acrylique superabsorbants, comme par exemple les produits commercialisés par la société BASF sous la dénomination LUQUASORB^{®} 1003 ou par la société CIBA Specialty Chemicals sous la dénomination SALCARE^{®} SC91 ainsi que les mélanges de ces composés.

La composition à base d'élastomère incluant des hydrocolloïdes peut inclure si nécessaire un ou plusieurs antioxydants, ainsi que le tensioactif MONTANOX^{®} 80 ou le polymère SEPINOV^{®} EMT 10 tous les deux commercialisés par la société SEPPIC pour optimiser la vitesse de gélification, la mouillabilité ou le relargage d'actifs éventuellement présents dans la composition.

Si l'on souhaite que la couche d'interface soit micro-adhérente ou adhérente, la composition à base d'élastomère incluant des hydrocolloïdes contient un produit tackifiant qui peut être choisi parmi les résines tackifiantes, les polyisobutylènes de bas poids moléculaire ou leurs mélanges. De façon générale, on préférera l'utilisation de résines hydrogénées telles que les résines ESCOREZ^{®} de la série 5000 et tout particulièrement la résine ESCOREZ^{®} 5380.

La composition peut contenir des principes actifs ayant un rôle favorable dans le traitement de la plaie. Parmi les substances susceptibles d'être utilisées dans le cadre de la présente invention on peut, à titre d'exemples, citer :
- les antibactériens comme par exemple les dérivés d'argent tels les sels d'argent ou d'autres métaux (par exemple le sulfate, le chlorure ou le nitrate d'argent et la sulfadiazine argentique), les complexes d'argent ou d'autres métaux (par exemple les zéolithes argent tel l'alphasan, ou les céramiques), le métrodinazole, la néomycine, le Polymyxine B, les pénicillines (Amoxycilline), l'acide clavulanique, les tétracyclines, la Minocycline, la chlorotétracycline, les aminoglycosides, l'Amikacine, la Gentamicine ou les probiotiques;
- les antiseptiques tels la chlorhexidine, le triclosan, le biguanide, l'hexamidine, le thymol, le Lugol, la Povidone iodée, le Chlorure de Benzalkonium et de Benzethonium;
- les anti-douleurs tels que le Paracétamol, la Codéine, le Dextropropoxyphène, le Tramadol, la Morphine et ses dérivés, les Corticoïdes et dérivés;
- les anti-inflammatoires tels que les Glucocorticoïdes, les anti-inflammatoires non stéroïdiens, l'Aspirine, l'Ibuprofène, le Kétoprofène, le Flurbiprofène, le Diclofénac, l'Acéclofénac, le Kétorolac, le Méloxicam, le Piroxicam, le Ténoxicam, le Naproxène, l'Indométacine, le Naproxcinod, le Nimésulide, le Célécoxib, l'Etoricoxib, le Parécoxib, le Rofécoxib, le Valdécoxib, la Phénylbutazone, l'acide niflumique, l'acide méfénamique;
- les actifs favorisant la cicatrisation tels que le Rétinol, la Vitamine A, la Vitamine E, la N-acétyl-hydroxyproline, les extraits de *Centella Asiatica,* la papaïne, les huiles essentielles de thym, de niaouli, de romarin et de sauge, l'acide hyaluronique, les oligosaccharides polysulfatés et leurs sels (en particulier les oligosaccharides sulfatés synthétiques ayant 1 à 4 unités oses tels que le sel de potassium du sucrose octasulfaté ou le sel d'argent du sucrose octasulfaté), le sucralfate, l'Allantoïne, l'urée, la metformine, les enzymes (par exemple protéolitiques telles la streptokinase, la tripsine ou la collagénase), des peptides ou des inhibiteurs de protéases;
- les anesthésiques tels que la benzocaïne, la lidocaïne, la dibucaïne, le chlorhydrate de pramoxine, la bupivacaïne, la mepivacaïne, la prilocaïne, ou l'étidocaïne.

On préférera employer des compositions micro-adhérentes à base d'élastomère contenant des hydrocolloïdes qui, pour un total de 100 % en poids, comprennent:
0,05 à 1 % en poids d'antioxydant ;
10 à 60 % en poids de résine tackifiante ;
2 à 20 %, de préférence de 12 à 16 %, en poids de carboxyméthylcellulose de sodium ;
10 à 65 % en poids d'une huile minérale plastifiante ;
5 à 25 % en poids d'un polymère tribloc poly(styrène-éthylène-butylène-styrène) ou poly(styrène-éthylène-propylène-styrène) ; et
1 à 15 % en poids d'un copolymère constitué d'un sel de l'acide 2-méthyl-2-[(1-oxo-2-propényl)amino]-1-propane-sulfonique et de l'ester 2-hydroxyéthyle de l'acide propénoïque.

Une autre composition élastomère hydrocolloïde pourra comprendre, pour un total de 100 % en poids:
0,05 à 1 % en poids d'antioxydant ;
2 à 20 %, de préférence de 12 à 16 %, en poids de carboxyméthylcellulose de sodium ;
20 à 65 % en poids d'une huile minérale plastifiante ; et
3 à 25 % en poids d'un polymère tribloc poly(styrène-éthylène-butylène-styrène) ou poly(styrène-éthylène-propylène-styrène).

La mousse absorbante ou la mousse absorbante recouverte d'une couche d'interface pourra être protégée, au moins sur sa face destinée à venir en contact avec la plaie, d'une pellicule de protection qui pourra être retirée par pelage avant utilisation du pansement.

Le protecteur pelable 3 peut être constitué d'une ou plusieurs parties pelables avant usage. Ce protecteur recouvre toute la surface de la mousse absorbante ou l'ensemble de la surface du support, dans le cas où le pansement comprend un trottoir adhésivé.

Ce protecteur pourra être tout matériau couramment utilisé comme protecteur par l'homme du métier dans le domaine des pansements. Il pourra se présenter par exemple sous forme de film par exemple un film de polyoléfine tels que le polyéthylène ou le polypropylène, un film de polyester mais également un papier. Ce film est avantageusement traité sur l'une au moins de ses faces par un composé siliconé comme un silane, un composé fluoré, ou un composé siliconé et fluoré.

Ce protecteur devra être adapté à la nature micro-adhérente de la couche d'interface. Ce protecteur devra également, le cas échéant, être adapté à la nature de gel de silicone adhésif utilisé pour le support s'il comprend un trottoir.

Dans le cadre de la présente invention, on préférera en particulier l'utilisation d'un protecteur en deux parties, comme indiqué sur les figures.

Le protecteur pelable est de préférence doté d'une épaisseur comprise entre 10 et 100 µm, par exemple de l'ordre de 50 µm.

Le produit commercialisé sous la référence Silflu^{®} M1R88001 par la société Siliconature peut avantageusement être utilisé comme tel.

Le pansement de l'invention est avantageusement indiqué pour le traitement de toutes les plaies exsudatives chroniques (escarre, ulcère, comme par exemple un ulcère du pied du diabétique) et aiguës (brûlure du deuxième degré, dermabrasion, plaie traumatique, plaie post-opératoire).

Le pansement de l'invention, lorsqu'il comprend un trottoir adhésif, est particulièrement indiqué pour le traitement des plaies lorsque la peau périlésionnelle est fragilisée.

Dans le cadre de la présente invention, on préférera l'utilisation d'un pansement à coins arrondis pour éviter un décollement prématuré.

Le pansement de l'invention peut se présenter sous forme de pansements individuels de petite dimension ou de dimension plus importante. Les pansements seront conditionnés individuellement dans une enveloppe scellée assurant une conservation en milieu stérile.

La présente invention a également pour objet un procédé de fabrication du pansement décrit précédemment qui consiste à réaliser le support et à assembler le support à la mousse absorbante. Selon un mode de réalisation, la mousse absorbante est fixée au voile non absorbant, puis l'ensemble est assemblé au support en mettant en contact le voile non absorbant et le côté du support recouvert de gel de silicone adhésif. Par exemple, la mousse absorbante et le voile non absorbant sont thermoscellés avant d'être calandrés au support.

Dans une première étape, on réalise le support par enduction de l'armature et assemblage de l'armature enduite au film continu.

Le gel de silicone sera enduit sur l'armature en utilisant une des techniques d'enduction couramment employée par l'homme de l'art.

Selon la version préférée de la présente invention, le gel de silicone est enduit sur les deux faces de l'armature, qui est assemblée au film continu alors que la réticulation du gel n'est pas complète, de manière à assurer la cohésion entre le film et l'armature ajourée. Dans ce mode de mise en oeuvre, aucun adhésif n'est nécessaire pour faire adhérer l'armature au film imperméable aux liquides et perméable à la vapeur d'eau.

Dans cette version, le support peut être fabriqué selon la succession d'étapes suivantes :
- l'armature est recouverte sur ses deux faces d'un mélange des précurseurs du gel de silicone,
- l'armature est assemblée au film par exemple par calandrage, et
- la réticulation du gel de silicone est provoquée ou accélérée une fois que l'armature et le film ont été assemblés, en plaçant par exemple le support dans un four.

L'armature est par exemple plongée dans le mélange des précurseurs du gel de silicone, puis essorée dans un poste de laminage entre deux rouleaux. Une soufflerie permet de reconstituer les ouvertures de l'armature pour enlever le surplus de gel de silicone.

Dans une autre version, le gel de silicone est enduit sur une des deux faces de l'armature, et l'autre face est fixée au film continu par un adhésif. Elle peut aussi être fixée par la chaleur, les ultra-sons ou la haute fréquence et dans ce cas, l'armature et/ou le film continu pourront être thermoplastiques, de manière à les thermosceller.

Le support pourra être protégé en recouvrant sa face adhésive par une couche ou pellicule de protection. Cette couche de protection pourra être par exemple du papier ou un film de polyester.

Dans une deuxième étape, le voile non absorbant est fixé à la mousse absorbante. Une bobine de mousse et une bobine de voile non absorbant sont déroulées puis détendues, avant d'être superposées. Le voile et la mousse sont fixés ensemble seulement sur leur périphérie par la chaleur, les ultrasons, la haute fréquence ou avec un adhésif. On procède par exemple au thermoscellage sur toute la périphérie des morceaux de mousse, de préférence par la chaleur ou la haute fréquence. La température de scellage sera par exemple comprise entre 80 et 150°C, de préférence encore entre 90 et 120 °C, notamment de l'ordre de 110°C. On procède à la fixation du voile et de la mousse, de préférence sur la périphérie de la mousse seulement, par exemple sur une largeur de 1 à 3 mm. On procède ensuite à un échenillage, qui permet de découper de façon sélective la mousse dans son épaisseur sans découper le voile, de préférence sous forme de carrés.

La mousse fixée au voile est ensuite assemblée au support fabriqué précédemment dans un poste de laminage, en appliquant une pression de consigne allant de 0 à 10 bars, de préférence de 0 à 6 bars. Le poste de laminage est avantageusement un poste de tirage.

Avant de réaliser la découpe, on recouvre l'ensemble support/voile/mousse, du côté de la mousse absorbante destinée à venir en contact avec la plaie, avec une pellicule de protection qui pourra être retirée par pelage avant utilisation du pansement.

Si la couche absorbante a été préalablement recouverte d'une couche d'interface et d'un protecteur pelable provisoire, ledit protecteur provisoire sera retiré pour recouvrir l'ensemble support/voile/mousse/couche d'interface, du côté de la couche d'interface destinée à venir en contact avec la plaie, avec une pellicule de protection qui pourra être retirée par pelage avant utilisation du pansement.

Lorsque l'on souhaite fixer le voile non absorbant à la mousse avec un adhésif, le voile non absorbant déroulé et détendu est enduit d'adhésif avant d'être calandré avec la couche de mousse. On procède ensuite à un échenillage, puis à la fixation de l'ensemble au support dans les mêmes conditions que celles décrites précédemment.

Selon un mode de mise en oeuvre, lorsque le pansement comprend un non tissé absorbant, on procèdera à la superposition des matériaux déroulés et détendus provenant d'une bobine de mousse, d'une bobine de voile non absorbant et d'une bobine de non tissé absorbant. L'ensemble des trois couches subira deux étapes d'échenillage successives afin de couper le non tissé absorbant à une dimension plus petite que celle de la mousse. Si le non tissé comprend des particules de polymère super-absorbant, on préfère thermosceller le voile et la mousse absorbante par la chaleur.

Une fois le voile et la mousse absorbante fixés l'un à l'autre, l'ensemble est de préférence assemblé au support par le même procédé de laminage que celui décrit précédemment. Le choix d'un voile non absorbant conformément à la présente invention permet avantageusement d'assembler le support et le voile sans qu'il soit nécessaire d'avoir recours à un adhésif acrylique comme dans l'art antérieur. Le support et le voile non absorbant restent solidaires lorsque le pansement usagé est retiré, ou décollé de la peau quand il comprend un trottoir adhésif.

Le voile et la mousse absorbante sont fixés l'un à l'autre de préférence au cours d'une étape qui précède la fixation du voile au support, afin d'éviter de fragiliser le gel de silicone.

Selon un mode de réalisation, le voile non absorbant subit avantageusement un traitement Corona, de préférence juste avant d'être fixé à la mousse absorbante. Le traitement Corona permet d'augmenter l'accroche du voile sur le support enduit de gel de silicone adhésif. Dans ce mode de réalisation, le voile peut être constitué de fibres de polyéthylène.

La mousse absorbante peut être recouverte d'une couche d'interface, de préférence avant d'être fixé au voile non absorbant.

Par exemple, la couche d'interface peut comprendre une composition à base d'élastomère et contenir des hydrocolloïdes telle que décrite précédemment. Cette composition peut être fabriquée suivant un procédé hot melt bien connu de l'homme du métier, par malaxage à chaud des différents constituants à une température comprise entre 90 et 160°C et de préférence entre 110 et 140°C. On préfère tremper un cylindre gravé dans la composition préalablement malaxée à chaud, puis la composition encore chaude est démoulée et transférée sur la mousse absorbante. L'application de la composition encore chaude sur la mousse permet d'optimiser l'accroche de la couche d'interface. Un protecteur provisoire est ensuite placé sur la mousse recouverte de la couche d'interface avant de procéder à l'étape d'échenillage. Le protecteur provisoire est retiré après la fixation de l'ensemble mousse/voile au support, pour appliquer un protecteur pelable qui sera retiré avant l'application du pansement sur la plaie.

L'invention est illustrée par l'exemple suivant.

### Exemple 1: Préparation d'un pansement absorbant

On fabrique un pansement comprenant une couche d'interface micro-adhérente, une mousse absorbante, un non tissé airlaid, un voile non absorbant de polyéthylène, un support enduit de gel de silicone adhésif et un protecteur pelable.

Les matériaux suivants sont utilisés :
- Le support est un tricot de polyester 40 g/m² enduit sur ses deux faces et sur toute sa surface d'un gel de silicone adhésif (200 g/m²), qui a été laminé sur un film de polyuréthane de 30 µm d'épaisseur. Ce support présente une épaisseur de l'ordre de 300 µm et une MVTR de l'ordre de 5000 g/m²/24 heures.
- Le voile non absorbant est par exemple un non tissé polyéthylène de 40 g/m² vendu sous la référence Vilmed^{®} LSO 1040 WEISS par Freudenberg.
- La mousse absorbante est une mousse de polyuréthane hydrophile de 3 mm vendue par CORPURA sous la référence MCF 03.
- Un non tissé absorbant est inséré entre le voile et la mousse : c'est un non tissé airlaid (200 g/m²) contenant un polymère super-absorbant de chez EAM Corporation vendu sous la référence Novathin^{®}.
- Le protecteur pelable de 50 microns d'épaisseur en PET silico-fluoré est formé de deux ailettes ; il est fourni par SILICONATURE sous la référence SILFLU^{®} M1R88001.

### Préparation de la couche d'interface et enduction sur la mousse absorbante :

On prépare la composition suivante, exprimée en pourcentage en poids par rapport au poids total:
- Huile minérale commercialisée par la société Shell sous la dénomination Ondina ^{®}919 : 39,7%
- Sel de sodium de carboxyméthylcellulose commercialisé par la société AQUALON sous la dénomination CMC Blanose ^{®} 7H4XF : 14,8%
- Copolymère séquencé de poly(styrène-éthylène-butylène) commercialisé par la société KRATON sous la dénomination KRATON^{®} G 1651E : 4,7%
- Antioxydant commercialisé sous la dénomination IRGANOX^{®} 1010 par la société CIBA SPECIALTY CHEMICALS : 0,2%
- Copolymère de sel de l'acide 2-méthyl-2[(1-oxo-2-propényl)amino]-1-propanesulfonique et de l'ester 2-hydroxyéthyle de l'acide propénoïque (agent de relargage) commercialisé par la société SEPPIC sous la dénomination SEPINOV^{®} EMT 10 : 5%
- Résine tackifiante commercialisée par la société EXXON CHEMICALS sous la dénomination ESCOREZ^{®} 5380 : 35,6%.

On a introduit l'huile minérale, l'hydrocolloïde, et l'élastomère puis l'antioxydant et l'agent de relargage et enfin la résine tackifiante portés à une température comprise entre 100 et 110°C dans un malaxeur MEL G-40, de manière à obtenir un mélange homogène.

Le mélange précédent est enduit de façon discontinue en une quantité de 170 g/m² (± 40) sur la mousse polyuréthane hydrophile.

### Assemblage des couches :

- La feuille de voile de polyéthylène subit un traitement Corona dans les conditions suivantes.
   - Puissance du générateur : 570 watts
   - Nombre d'électrodes/largeur : 3/0,25 m
   - Réglage de l'entrefer : 2 mm
   - Vitesse de défilement : 2 m/minute
- Avant que les effets du traitement Corona aient disparu, on soude sur une largeur de 2 mm avec une soudeuse manuelle AMIS sur un seul côté le voile et la mousse de polyuréthane hydrophile ; le non tissé absorbant est ensuite inséré entre la mousse et le voile.
- Les trois derniers scellages sont effectués dans les mêmes conditions que précédemment de manière à former un carré de 8 x 8 cm, puis les bords du complexe assemblé sont découpés.
- Le support est découpé selon un carré de 15 x 15 cm, puis assemblé au complexe précédant par calandrage avec un rouleau de 10 kg, dans deux directions perpendiculaires.
- On procède ensuite à la découpe du pansement final 13 x 13 cm, en arrondissant les coins.

## Revendications

1. Pansement comprenant une mousse absorbante (2) et un support de protection imperméable aux fluides et perméable à la vapeur d'eau (4),
- ledit support (4) étant constitué par l'assemblage d'un film continu (4a) et d'une armature ajourée enduite, sur au moins une de ses faces, d'un gel de silicone adhésif (4b), sans obturer les ouvertures de l'armature, ladite armature recouvrant l'intégralité de la surface du film,
- ledit pansement comprenant en outre un voile non absorbant (5) intercalé entre ladite mousse absorbante (2) et ledit support, lequel voile colle au gel de silicone adhésif (4b) enduit sur ladite armature, et fixé à la mousse absorbante (2).

2. Pansement selon l'une des revendications précédentes, **caractérisé en ce que** le voile non absorbant (5) présente un grammage compris entre 15 et 50 g/m².

3. Pansement selon l'une des revendications précédentes, **caractérisé en ce que** le voile non absorbant (5) comprend des fibres de polyéthylène.

4. Pansement selon l'une des revendications précédentes, **caractérisé en ce que** la mousse absorbante (2) est centrée sur le support (4) dont la surface est supérieure, de manière à créer des bordures adhésives.

5. Pansement selon l'une des revendications précédentes, **caractérisé en ce que** la mousse absorbante (2) est une mousse de polyuréthane hydrophile.

6. Pansement selon l'une des revendications précédentes, **caractérisé en ce que** le grammage du gel de silicone adhésif est compris entre 100 et 500 g/m².

7. Pansement selon l'une des revendications précédentes, **caractérisé en ce que** la mousse absorbante (2) est recouverte, sur la surface faisant face à la plaie, d'une couche d'interface discontinue (1) comprenant un élastomère, un plastifiant et des hydrocolloïdes.

8. Pansement selon l'une des revendications précédentes, **caractérisé en ce que** l'armature ajourée (4b) est un tricot enduit de gel de silicone adhésif sur ses deux faces et sur toute sa surface sans obturer les ouvertures du tricot.

9. Pansement selon l'une des revendications 1 à 7, **caractérisé en ce que** l'armature ajourée (4b) est un film perforé enduit de gel de silicone adhésif sur une de ses surfaces, sans obturer les perforations du film.

10. Pansement selon l'une des revendications précédentes, pour son application sur une plaie, telles que les plaies exsudatives chroniques, notamment une escarre, un ulcère, comme l'ulcère du pied du diabétique, et les plaies aiguës, telles qu'une brûlure du deuxième degré, une dermabrasion, une plaie traumatique ou une plaie post-opératoire.

11. Pansement selon la revendication 1, **caractérisé en ce qu'**il comprend en outre un non tissé absorbant (6) placé entre la mousse absorbante (2) et le voile (5).

12. Procédé de fabrication d'un pansement selon l'une des revendications précédentes consistant à réaliser le support (4), à fixer le voile non absorbant (5) et la mousse (2), puis à assembler l'ensemble au support (4).

13. Procédé selon la revendication 12, **caractérisé en ce que** le voile et (5) la mousse (2) sont fixés ensemble seulement sur leur périphérie par la chaleur, les ultrasons, la haute fréquence ou avec un adhésif.

14. Procédé selon la revendication 12 ou 13, **caractérisé en ce que** le support (4) est assemblé à l'ensemble constitué du voile (5) et de la mousse (2) dans un poste de laminage en appliquant une pression allant de 0 à 10 bars.

15. Procédé selon l'une des revendications 12 à 14, **caractérisé en ce que** le voile non absorbant (5) subit un traitement Corona, juste avant d'être fixé à la mousse.

## Patentansprüche

1. Verband, umfassend einen absorbierenden Schaum (2) und einen flüssigkeitsundurchlässigen und wasserdampfdurchlässigen Schutzträger (4),
- wobei der Träger (4) durch Zusammenfügen eines durchgehenden Films (4a) und einer durchbrochenen Einlage, die auf wenigstens einer ihrer Seiten mit einem Haftsilikongel (4b) überzogen ist, ohne die Öffnungen der Einlage zu verschließen, gebildet ist, wobei die Einlage die gesamte Oberfläche des Films bedeckt,
- wobei der Verband ferner einen nicht absorbierenden Flor (5) umfasst, der zwischen dem absorbierenden Schaum (2) und dem Träger eingefügt ist, welcher Flor an dem auf die Einlage aufgebrachten Haftsilikongel (4b) klebt, und an dem absorbierenden Schaum (2) fixiert ist.

2. Verband nach dem vorhergehenden Anspruch, **dadurch gekennzeichnet, dass** der nicht absorbierende Flor (5) ein Flächengewicht zwischen 15 und 50 g/m² aufweist.

3. Verband nach einem der vorhergehenden Ansprüche, **dadurch gekennzeichnet, dass** der nicht absorbierende Flor (5) Polyethylenfasern umfasst.

4. Verband nach einem der vorhergehenden Ansprüche, **dadurch gekennzeichnet, dass** der absorbierende Schaum (2) auf dem Träger (4), dessen Fläche größer ist, zentriert ist, um Haftumrandungen zu schaffen.

5. Verband nach einem der vorhergehenden Ansprüche, **dadurch gekennzeichnet, dass** der absorbierende Schaum (2) ein hydrophiler Polyurethanschaum ist.

6. Verband nach einem der vorhergehenden Ansprüche, **dadurch gekennzeichnet, dass** das Flächengewicht des Haftsilikongels zwischen 100 und 500 g/m² beträgt.

7. Verband nach einem der vorhergehenden Ansprüche, **dadurch gekennzeichnet, dass** der absorbierende Schaum (2) auf der der Wunde gegenüberliegenden Fläche mit einer unterbrochenen Grenzflächenschicht (1), welche ein Elastomer, einen Weichmacher und Hydrokolloide umfasst, überzogen ist.

8. Verband nach einem der vorhergehenden Ansprüche, **dadurch gekennzeichnet, dass** die durchbrochene Einlage (4b) ein Gewirk ist, das auf seinen beiden Seiten und auf seiner gesamten Oberfläche mit Haftsilikongel überzogen ist, ohne die Öffnungen des Gewirks zu verschließen.

9. Verband nach einem der Ansprüche 1 bis 7, **dadurch gekennzeichnet, dass** die durchbrochene Einlage (4b) ein perforierter Film ist, der auf einer seiner Flächen mit Haftsilikongel überzogen ist, ohne die Löcher des Films zu verschließen

10. Verband nach einem der vorhergehenden Ansprüche, für seine Anwendung auf einer Wunde, wie chronischen exsudativen Wunden, insbesondere einem Dekubitus, einem Geschwür, wie dem diabetischen Fußgeschwür, und akuten Wunden, wie einer Verbrennung zweiten Grades, einer Dermabrasion, eine traumatischen Wunde oder einer postoperativen Wunde.

11. Verband nach Anspruch 1, **dadurch gekennzeichnet, dass** er ferner ein absorbierendes Vlies (6), das zwischen dem absorbierenden Schaum (2) und dem Flor (5) angeordnet ist, umfasst.

12. Verfahren zur Herstellung eines Verbands nach einem der vorhergehenden Ansprüche, das darin besteht, den Träger (4) herzustellen, den nicht absorbierenden Flor (5) und den Schaum (2) zu fixieren, anschließend das Ganze mit dem Träger (4) zusammenzufügen.

13. Verfahren nach Anspruch 12, **dadurch gekennzeichnet, dass** der Flor (5) und der Schaum (2) nur an ihrem Umfang durch Hitze, Ultraschall, Hochfrequenz oder mit einem Klebemittel fixiert werden.

14. Verfahren nach Anspruch 12 oder 13, **dadurch gekennzeichnet, dass** der Träger (4) mit der Anordnung bestehend aus dem Flor (5) und dem Schaum (2) in einer Walzstation unter Anlegen eines Druckes von 0 bis 10 Bar zusammengefügt wird.

15. Verfahren nach einem der Ansprüche 12 bis 14, **dadurch gekennzeichnet, dass** der nicht absorbierende Flor (5) direkt vor seiner Fixierung an dem Schaum einer Corona-Behandlung unterzogen wird.

## Claims

1. A dressing comprising an absorbent foam (2) and a protective substrate (4) that is impermeable to fluids, but permeable to water vapor,
- said substrate (4) being formed by assembling a continuous film (4a) and an openwork reinforcement that is coated, on at least one of its faces, with an adhesive silicone gel (4b) without blocking the openings in the reinforcement, said reinforcement covering the entire surface of the film,
- said dressing also comprising a non-absorbent web (5) which is inserted between said absorbent foam (2) and said substrate, which web adheres to the adhesive silicone gel (4b) that is coated onto said reinforcement, and which is secured to the absorbent foam (2).

2. The dressing as claimed in the preceding claim, **characterized in that** the non-absorbent web (5) has a grammage of between 15 and 50 g/m².

3. The dressing as claimed in either of the preceding claims, **characterized in that** the non-absorbent web (5) comprises polyethylene fibers.

4. The dressing as claimed in one of the preceding claims, **characterized in that** the absorbent foam (2) is centered on the substrate (4), the surface of which is greater, so as to create adhesive borders.

5. The dressing as claimed in one of the preceding claims, **characterized in that** the absorbent foam (2) is a hydrophilic polyurethane foam.

6. The dressing as claimed in one of the preceding claims, **characterized in that** the grammage of the adhesive silicone gel is between 100 and 500 g/m².

7. The dressing as claimed in one of the preceding claims, **characterized in that** the absorbent foam (2) is covered, on the surface facing the wound, with a discontinuous interface layer (1) comprising an elastomer, a plasticizer and hydrocolloids.

8. The dressing as claimed in one of the preceding claims, **characterized in that** the openwork reinforcement (4b) is a knit that is coated with adhesive silicone gel on both its faces and on its entire surface without blocking the openings of the knit.

9. The dressing as claimed in one of claims 1 to 7, **characterized in that** the openwork reinforcement (4b) is a perforated film that is coated with adhesive silicone gel on one of its faces, without blocking the perforations of the film.

10. The dressing as claimed in one of the preceding claims, for use on a wound, such as exudative chronic wounds, in particular an eschar, an ulcer, for instance a diabetic's foot ulcer, and acute wounds, such as a second-degree burn, a dermabrasion, a trauma wound or a post-operative wound.

11. The dressing as claimed in claim 1, **characterized in that** an absorbent nonwoven is placed between the absorbent foam (2) and the web (5).

12. A process for manufacturing a dressing as claimed in one of the preceding claims, which consists in producing the substrate (4), in securing the non-absorbent web (5) and the foam (2), and then in assembling the assembly to the substrate (4).

13. The process as claimed in claim 12, **characterized in that** the web (5) and the foam (2) are secured together only along their periphery via heat, ultrasound, high frequency or with an adhesive.

14. The process as claimed in claim 12 or 13, **characterized in that** the substrate (4) is assembled to the assembly consisting of the web (5) and the foam (2) in a lamination station by applying a pressure ranging from 0 to 10 bar.

15. The process as claimed in one of claims 12 to 14, **characterized in that** the non-absorbent web (5) undergoes a Corona treatment, just before being secured to the foam.
